# EUROPÄISCHE PATENTSCHRIFT

(11) **EP 1 164 917 B1**
(45) Veröffentlichungstag und Bekanntmachung des Hinweises auf die Patenterteilung: **18.10.2006**
(21) Anmeldenummer: 01911564.1
(22) Anmeldetag: 31.01.2001
(51) Int. Cl.: A61B 1/313, A61B 1/05

(54) **VORRICHTUNG ZUR INTRAKORPORALEN, MINIMAL-INVASIVEN BEHANDLUNG EINES PATIENTEN**
DEVICE FOR INTRACORPOREAL, MINIMAL INVASIVE TREATMENT OF A PATIENT
DISPOSITIF PERMETTANT DE TRAITER UN PATIENT DE MANIERE INTRACORPORELLE ET A INVASION MINIMALE

(30) Priorität: 01.02.2000 DE 10004264
(43) Veröffentlichungstag der Anmeldung: 02.01.2002
(73) Patentinhaber: Karl Storz GmbH & Co. KG, 78532 Tuttlingen (DE)
(72) Erfinder: IRION, Klaus, M., 78576 Liptingen (DE); SCHWARZ, Peter, 78532 Tuttlingen (DE); KOCHER, Mark, 71065 Sindelfingen (DE)
(74) Vertreter: Heuckeroth, Volker
(86) Internationale Anmeldenummer: PCT/EP2001/001039
(87) Internationale Veröffentlichungsnummer: WO 2001/056460

(56) Entgegenhaltungen:
- WO-A-98/46120
- US-A- 5 166 787
- US-A- 5 368 015
- US-A- 5 408 409
- US-A- 5 825 982
- PATENT ABSTRACTS OF JAPAN vol. 1997, no. 06, 30. Juni 1997 (1997-06-30) & JP 09 028663 A (OLYMPUS OPTICAL CO LTD), 4. Februar 1997 (1997-02-04)

## Beschreibung

Die vorliegende Erfindung betrifft eine Vorrichtung zur intrakorporalen, minimal-invasiven Behandlung eines Patienten, mit einem Arbeitsinstrument, das zur Durchführung eines Behandlungsschrittes in einen Körperhohlraum eines Patienten einführbar ist, wobei ein distales Ende des eingeführten Arbeitsinstruments einen intrakorporalen Arbeitsbereich definiert, und mit zumindest einer Bildaufnahmeeinheit zum Aufnehmen eines Abbildes des intrakorporalen Arbeitsbereichs, ferner mit Positioniermitteln zum Ausrichten einer optischen Achse der Bildaufnahmeeinheit in Abhängigkeit von einer räumlichen Lage des intrakorporalen Arbeitsbereichs, wobei die Positioniermittel einen Führungsschaft beinhalten, in dem das Arbeitsinstrument geführt ist, und wobei die Bildaufnahmeeinheit verschwenkbar an einem intrakorporalen Abschnitt des Führungsschaftes befestigt ist.

Eine solche Vorrichtung ist aus der US 5,166,787 bekannt.

Bei der minimal-invasiven, chirurgischen Behandlung von Patienten werden Arbeitsinstrumente nur noch durch eine oder mehrere kleine Einschnittsöffnungen an die zu behandelnde Stelle im Körper des Patienten herangebracht. Die visuelle Kontrolle der im Körperinneren durchzuführenden Arbeitsschritte erfolgt endoskopisch. Ein Beispiel für eine minimal-invasive Standardoperation ist die laparoskopische Colecystectomie. Hierbei werden drei kleine Öffnungen in den Bauchraum des Patienten geschnitten. Eine der Öffnungen dient zum Einführen eines Endoskops mit einer Videokamera, deren Bild für den behandelnden Arzt auf einem Monitor sichtbar ist. Durch die beiden anderen Öffnungen werden Arbeitsinstrumente, wie beispielsweise Scheren, Zangen, etc. eingeführt.

Bei der Durchführung der Operation wird das Endoskop heutzutage üblicherweise von einem Assistenzarzt bedient und Veränderungen und Verschiebungen des Arbeitsbereichs soweit nachgeführt, daß der behandelnde Arzt die Arbeitsinstrumente stets im Blickfeld behält. Eine solche Kameraassistenz erfordert jedoch eine sehr gute Abstimmung zwischen dem behandelnden Arzt und dem Assistenzarzt, was im praktischen Einsatz häufig problematisch ist. Darüber hinaus vergrößert die erforderliche Kameraassistenz den Personalaufwand bei der Durchführung der Operation, was sich nachteilig auf die Kosten auswirkt.

In der DE 195 29 950 C1 wurde daher eine gattungsgemäße Vorrichtung vorgeschlagen, bei der der Kameraassistent durch einen außerhalb des Körpers des Patienten angeordneten, automatisch gesteuerten Roboter ersetzt wird. Zur Steuerung des Roboters wird das von der Endoskopkamera aufgenommene Bild in Bezug auf die Position des oder der Arbeitsinstrumente in dem aufgenommenen Bild ausgewertet. Die Arbeitsinstrumente sind hierzu farblich markiert, so daß sie mit Hilfe des vorgeschlagenen Bildverarbeitungsalgorithmus identifiziert werden können.

Eine solche Robotersteuerung kann zwar grundsätzlich den bislang erforderlichen Assistenzarzt ersetzen, sie ist jedoch in technischer Hinsicht aufwendig und besitzt zudem weitere Nachteile. Insbesondere benötigt das Robotersystem eine sehr große mechanische Halterung, die vergleichsweise viel Platz über dem Patienten einnimmt. Dies schränkt die Bewegungsfreiheit für den behandelnden Arzt über dem Patienten ein. Zudem ist die Sterilisierung der vergleichsweise großen Robotervorrichtung schwierig. Auch der Auf- und Abbau einer solchen Vorrichtung benötigt relativ viel Zeit, was sich besonderes bei Standardoperationen nachteilig auf die Kosten und die Effizienz auswirkt.

Aus der eingangs genannten US 5,166,787 ist ein Endoskop mit einer am distalen Ende angeordneten Videokamera bekannt, wobei die Videokamera nach dem Einführen in den zu beobachtenden Körperhohlraum des Patienten als Ganzes gegenüber dem distalen Ende des Endoskop-Schaftes bewegbar ist. Die Videokamera läßt sich um eine parallel zur Längsachse des Schafts verlaufende Achse, die eine körpereigene Achse der Videokamera bildet, in einer Ebene quer zur Längsrichtung des Schaftes aus der Längsachse des Schaftes heraus verschwenken. Die Blickrichtung der Videokamera bleibt dabei in jeder Schwenkstellung der Videokamera parallel zur Längsmittelachse des Schaftes. In einem weiteren Ausführungsbeispiel ist die Videokamera zusätzlich noch um eine quer zur Längsrichtung des Schaftes verlaufende Schwenkachse wiederum um eine Videokamera-eigene Achse verschwenkbar. Hierbei ändert sich zwar der Blickwinkel der Videokamera bezüglich der Längsachse des Schafts, jedoch werden dabei nur Blickrichtungen erzeugt, die mit der Längsachse des Schaftes einen sehr spitzen Winkel bilden, oder solche, die von der Längsmittelachse des Schaftes abgewandt sind und daher bei einem Einsatz des Endoskopes bei einer Operation nicht sinnvoll sind. Mit anderen Worten, können mit diesem bekannten Endoskop nicht die gleichen oder zumindest ähnlichen perspektivischen Beobachtungsverhältnisse wie bei einem über einen weiteren Zugang in den Körperhohlraum eingeführten Endoskop erreicht werden.

Weder das bekannte Endoskop noch die Videokamera weisen darüber hinaus solche Positioniermittel auf, mit denen die Blickrichtung bzw. das Bildfeld der Videokamera bestimmten Verschiebungen oder Verlagerungen des intrakorporalen Arbeitsbereichs automatisch nachgeführt werden kann. Es ist somit auch bei dieser bekannten Vorrichtung eine manuelle Positionierung mit den bereits erwähnten Nachteilen erforderlich.

Es ist daher eine Aufgabe der vorliegenden Erfindung, eine Vorrichtung der eingangs genannten Art anzugeben, mit der zumindest ähnliche perspektivische Sichtverhältnisse erreicht werden wie bei einem über einen weiteren Zugang in den Körperhohlraum eingeführten Endoskop.

Diese Aufgabe wird bei der eingangs genannten Vorrichtung erfindungsgemäß dadurch gelöst, daß die Positioniermittel eine am intrakorporalen Abschnitt des Führungsschafts verschwenkbar befestigte Halterung aufweisen, an der die Bildaufnahmeeinheit von einer Anlenkstelle der Halterung am Führungsschaft beabstandet angeordnet ist, derart, dass die Bildaufnahmeeinheit intrakorporal in eine Arbeitsposition schwenkbar ist, in der die optische Achse zu einer Längsmittelachse des Führungsschafts hinweisend winklig zu dieser verläuft.

Die Aufgabe wird des Weiteren durch einen Führungsschaft zur Verwendung in einer Vorrichtung zur intrakorporalen, minimalinvasiven Behandlung eines Patienten gelöst, wobei in die vorrichtung ein Arbeitsinstrument zur Durchführung eines Behandlungsschrittes in einem Körperhohlraum einführbar ist, und die Vorrichtung zumindest eine eine optische Achse aufweisende Bildaufnahmeeinheit aufweist, wobei der Führungsschaft einen intrakorporalen Abschnitt aufweist, der Positioniermittel zur verschwenkbaren Befestigung einer Bildaufnahmeeinheit aufweist, wobei der Führungsschaft dadurch gekennzeichnet ist, dass die Positioniermittel eine am intrakorporalen Abschnitt des Führungsschafts verschwenkbar befestigte Halterung aufweisen, an der die Bildaufnahmeeinheit von einer Anlenkstelle der Halterung am Führungsschaft beabstandet angeordnet ist, derart, dass die Bildaufnahmeeinheit intrakorporal in eine Arbeitsposition schwenkbar ist, in der die optische Achse zu einer Längsmittelachse des Führungssohaftes hinweisend winklig zu dieser verläuft.

Die erfindungsgemäße Vorrichtung unterscheidet sich von der bekannten Vorrichtung insbesondere dadurch, dass die Bildaufnahmeeinheit über eine Halterung am Führungsschaft befestigt, und durch die verschwenkbarkeit der Halterung vom Führungsschaft weg schwenkbar ist, d.h. vom Führungsschaft beabstandbar ist. Auf diese Weise kann die Blickrichtung unter einem größeren Winkel zur Längsachse des Führungsschafts positioniert werden, was den perspektivischen Sichtverhältnissen eines durch eine weitere Öffnung in den Körperhohlraum eingeführten Endoskops entspricht, was aus Sicht des Arztes erwünscht ist. Durch die Kopplung der Bildaufnahmeeinheit mit dem intrakorporalen Abschnitt des Führungsschaftes ist es darüber hinaus wie hiernach noch näher beschrieben wird, möglich, daß die Bildaufnahmeeinheit automatisch zumindest einen Teil der Bewegungen des Arbeitsinstruments folgen kann, ohne daß ein Roboter oder eine entsprechende Vorrichtung außerhalb des Körperhohlraums benötigt wird. Es handelt sich damit praktisch um ein sehendes Arbeitsinstrument. Die erfindungsgemäße Vorrichtung ist infolge dessen wesentlich kleiner und raumsparender und kostengünstiger als herkömmliche Vorrichtungen mit extrakorporaler Positioniereinrichtung.

Durch die vorliegende Erfindung wird praktisch ein "distales Ende eines Endoskops" an das Arbeitsinstrument angekoppelt, das mit perspektivischer Blickrichtung auf das Arbeitsgebiet positionierbar ist, so, wie wenn ein ganzes Endoskop durch eine weitere Öffnung in den Körperhohlraum eingeführt würde.

In bevorzugten Ausgestaltungen der Erfindung, die nachfolgend näher beschrieben werden, kann auf eine separate Halterung für die Bildaufnahmeeinheit außerhalb des Körperhohlraums des Patienten sogar vollständig verzichtet werden, so daß in diesem Fall überhaupt kein zusätzlicher Platz über dem Patienten erforderlich ist. Der Bewegungsfreiraum für den behandelnden Arzt ist dann überhaupt nicht mehr eingeschränkt.

Zusätzlich verringert sich bei der kleineren Vorrichtung der Aufwand für den Auf- und Abbau, was der Effizienz und Handhabung im praktischen Einsatz zu Gute kommt.

Schließlich ist auch die Sterilisierung der erfindungsgemäßen Vorrichtung aufgrund der geringeren Baugröße leichter. Trotz alledem besitzt die erfindungsgemäße Vorrichtung sämtliche Vorteile eines automatischen Nachführsystems, so daß insgesamt eine beträchtliche Kosteneinsparung bei minimal-invasiven Behandlungen von Patienten möglich ist. Außerdem wird das Risiko einer unbeabsichtigten Verschmutzung der Bildaufnahmeeinheit durch Gewebekontakt verringert. Ferner sind auch kürzere Operationszeiten infolge der verbesserten Handhabung erreichbar, was für den Patienten mit geringeren Belastungen und einem geringeren Operationsrisiko verbunden ist.

Bei minimal-invasiven Eingriffen werden, wie weiter oben bereits erwähnt, zwei Inzisionen geschaffen, wobei durch die erste, aus Sicht des Arztes üblicherweise die "rechte", ein aktives Arbeitselement, bspw. eine Schere, und durch die andere, aus Sicht des Arztes die "linke", ein passives oder passiveres Arbeitsinstrument, bspw. ein Halteinstrument, eingeführt wird. Bei der erfindungsgemäßen Vorrichtung ist es in diesem Sinne besonders bevorzugt, wenn die Bildaufnahmeeinheit über den Führungsschaft am passiveren Arbeitsinstrument angekoppelt ist.

Die zuvor genannte Aufgabe ist daher vollständig gelöst.

In einer bevorzugten Ausgestaltung weist die Halterung zumindest einen gelenkig mit dem intrakorporalen Abschnitt befestigten Schwenkarm auf.

Durch diese Maßnahme kann die Vorrichtung mit den zuvor genannten erfindungsgemäßen Funktionen und Wirkungen konstruktiv besonders einfach gestaltet werden, um eine Kopplung der Bildaufnahmeeinheit an dem Führungsschaft zur Erzielung perspektivischer Sichtverhältnisse auszugestalten.

In einer besonders einfachen bevorzugten Ausgestaltung ist die Bildaufnahmeeinheit am freien Ende des zumindest einen Schwenkarms so angeordnet, daß die optische Achse der Bildaufnahmeeinheit etwa senkrecht zur Längsachse des Schwenkarms verläuft und zur Längsmittelachse des Führungsschafts hinweist.

Bei dieser Ausgestaltung ist vorteilhafterweise nur ein Gelenk notwendig, nämlich dasjenige, über das der zumindest eine Schwenkarm mit dem Führungsschaft verbunden ist, und über das die Bildaufnahmeeinheit dann zur Einstellung eines perspektivischen Betrachtungswinkels vom Führungsschaft ausgeschwenkt werden kann, um den gewünschten perspektivischen Betrachtungswinkel zwischen der Längsachse des Führungsschafts oder Arbeitsinstruments und der Bildaufnahmeeinheit einzustellen.

Bei einer solchen einfachen Ausgestaltung der erfindungsgemäßen Vorrichtung ist es vorteilhaft, auch mehr als eine Bildaufnahmeeinheit zu integrieren, um ein Video-Stereosystem zur verbesserten räumlichen Abbildung und Wiedergabe zu erhalten.

Bei der zuvor genannten Ausgestaltung ist es weiterhin bevorzugt, wenn der Schwenkarm eine verstellbare Länge aufweist.

Diese Maßnahme hat den Vorteil, daß bei einer Verschiebung des Arbeitsinstruments in axialer Richtung der Winkel zwischen der optischen Achse und der Bildaufnahmeeinheit und der Längsachse des Arbeitsinstruments bei der zuvor genannten einachsigen Ausführung des Gelenkmechanismus durch Verkürzen oder Verlängern des Schwenkarmes konstant gehalten werden kann. Der Schwenkarm kann hierzu bspw. teleskopartig aufgebaut sein.

In einer weiteren bevorzugten Ausgestaltung der Erfindung weist die Halterung einen ein- oder mehrachsigen Gelenkmechanismus auf.

Diese Maßnahme besitzt den Vorteil, daß mit zunehmender Anzahl der Achsen des Gelenkmechanismus die Zahl der Freiheitsgrade der Bildaufnahmeeinheit in Bezug auf den Führungsschaft steigt. Infolgedessen kann die Bildaufnahmeeinheit bei Verwendung eines mehrachsigen Gelenkmechanismus optimal auf den Arbeitsbereich ausgerichtet werden.

In einer weiteren bevorzugten Ausgestaltung ist die Bildaufnahmeeinheit an dem Führungsschaft in eine Ruheposition schwenkbar, in der eine äußere Querschnittskontur der Bildaufnahmeeinheit im wesentlichen deckungsgleich zu einer äußeren Querschnittskontur des Führungsschaftes angeordnet ist.

Diese Maßnahme besitzt den Vorteil, daß die Bildaufnahmeeinheit zusammen mit dem Führungsschaft, d.h. durch dieselbe Öffnung, in den Körperhohlraum des Patienten eingeführt werden kann. Infolgedessen kann beispielsweise bei der laparoskopischen Colecystectomie auf einen der drei bisher benötigten Einschnitte verzichtet werden. Dies ist für den Patienten mit einer geringeren Traumatisierung verbunden, was wiederum ein geringeres Risiko zur Folge hat. Bei anderen Applikationen, wie zum Beispiel der Tubensterilisation ist mit dem erfindungsgemäßen perspektivisch sehenden Instrument sogar nur ein einziger Einschnitt erforderlich.

In einer weiteren Ausgestaltung der Erfindung ist die Bildaufnahmeeinheit über einen intrakorporal aktivierbaren Koppelmechanismus an dem Führungsschaft fixierbar.

Bei dieser Ausgestaltung der Erfindung kann die zunächst separate Bildaufnahmeeinheit intrakorporal an dem Führungsschaft befestigt werden. Hierdurch ist es möglich, die Bildaufnahmeeinheit getrennt von dem Führungsschaft in den Körperhohlraum des Patienten einzuführen, beispielsweise also über eine eigene Einschnittsöffnung. Die Maßnahme besitzt den Vorteil, daß sowohl die Bildaufnahmeeinheit als auch der Führungsschaft jeweils für sich genommen größer realisiert werden können, so daß insgesamt mehr Bauraum zur Verfügung steht. Dies ist insbesondere im Hinblick auf die Bildaufnahmeeinheit von Vorteil, da ein größerer Bauraum beispielsweise mehr Lichtleiter aufnehmen kann und daher eine höhere Lichtstärke ermöglicht.

In einer weiteren Ausgestaltung der Erfindung beinhalten die Positioniermittel eine mechanische Zwangskopplung zwischen dem Arbeitsinstrument und der Bildaufnahmeeinheit.

Eine mechanische Zwangskopplung ermöglicht auf einfache Weise ein automatisches Nachführen der Bildaufnahmeeinheit, ohne daß zusätzliche Stellantriebe oder Sensoren erforderlich sind. Infolgedessen kann diese Ausgestaltung der Erfindung sehr kostengünstig und außerdem sehr robust realisiert werden. Letzteres ist vor allem für den praktischen Einsatz im Alltag und beim Sterilisieren von Vorteil.

Dabei ist es bevorzugt, wenn die Positioniermittel Arretiermittel zum zumindest teilweisen axialen Festlegen des Arbeitsinstruments relativ zum Führungsschaft aufweisen.

Diese Maßnahme stellt die einfachste Nachführung zwischen dem Bildaufnahmesystem und dem Arbeitsbereich dar, indem die über die Halterung mit dem Führungsschaft verbundene Bildaufnahmeeinheit durch die axiale Fixierung des Arbeitsinstruments relativ zum Führungsschaft bei jeder axialen Verschiebung des Arbeitsinstruments mitgenommen wird. Die Verschwenkung bzw. Auslenkung der Halterung bleibt dabei im einfachsten Fall erhalten, so daß auch die perspektivische Blickrichtung unverändert bleibt. Der perspektivische Betrachtungswinkel kann zuvor durch Verschwenken der Halterung und damit der Bildaufnahmeeinheit so fixiert werden, daß die Spitze des Arbeitsinstruments, die den Arbeitsbereich definiert, ungefähr in der Bildmitte zum liegen kommt. Das Arbeitsinstrument wird dann bspw. proximalseitig so an dem Führungsschaft mittels der Arretiermittel festgelegt, daß es zwar vorzugsweise rotatorisch noch im Führungsschaft beweglich ist, jedoch axial sich nur in Verbindung mit dem Führungsschaft bewegen läßt. Um eine vollständige axiale Fixierung des Arbeitsinstruments am Führungsschaft zu erreichen, kann im einfachsten Fall im Führungsschaft eine Ringnut vorgesehen sein, in die ein am Arbeitsinstrument befindlicher, vorzugsweise gefederter Zapfen läuft. Durch eine solche Arretierung ist gewährleistet, daß der Arbeitsbereich trotz einer Bewegung des Arbeitsinstruments im Bildbereich der Bildaufnahmeeinheit liegt.

Dabei ist es weiterhin bevorzugt, wenn die Arretiermittel so ausgebildet sind, daß das Arbeitsinstrument innerhalb vorbestimmter Grenzen axial relativ zum Führungsschaft frei schiebbar ist, bei einer über die vorbestimmten axialen Grenzen hinausgehenden Verschiebung den Führungsschaft jedoch axial mitnimmt.

Da es unter Umständen als störend empfunden werden kann, daß die Bildaufnahmeeinheit sich stets mitbewegt, wenn das Arbeitsinstrument axial verschoben wird, so daß keine visuelle Registrierung der Instrumentenbewegung möglich ist. Durch die zuvor beschriebene Ausgestaltung ist es nunmehr möglich, das Instrument axial relativ zum Führungsschaft innerhalb vorbestimmter Grenzen zu bewegen, ohne daß der Führungsschaft und damit die Bildaufnahmeeinheit mitbewegt wird. Die zuvor genannten Grenzen werden vorzugsweise so eingestellt, daß sie gerade der Strecke zwischen dem Ein- und Austreten der Spitze des Arbeitsinstruments aus dem Bildfeld entspricht. Erst wenn die Spitze des Arbeitsinstruments aus dem Bildfeld herauswandern würde, werden die Arretiermittel aktiv und bewegen dann den Führungsschaft und damit die Bildaufnahmeeinheit mit. Derartige Arretiermittel können durch eine breitere Ringnut im Führungsschaft realisiert werden, in der ein gegenüber der axialen Länge der Ringnut axial kürzerer Zapfen am Arbeitsinstrument läuft.

Gegenüber den zuvor genannten konstruktiv sehr einfachen Nachführungen ist es ebenso bevorzugt, wenn das Arbeitsinstrument relativ zum Führungsschaft axial frei verschiebbar ist, und daß die Halterung Koppelmittel aufweist, die mit dem Arbeitsinstrument in Eingriff bringbar sind, derart, daß bei einer Relativverschiebung zwischen Arbeitsinstrument und Führungsschaft die Halterung verschwenkt wird, um die optische Achse dem Arbeitsbereich nachzuführen.

Bei dieser Ausgestaltung führt demnach eine axiale Relativverschiebung zwischen dem Arbeitsinstrument und dem Führungsschaft zu einer Verschwenkung der Halterung am Führungsschaft und damit zu einer Änderung der Blickrichtung der Bildaufnahmeeinheit, wobei die Kopplung bewirkt, daß die Blickrichtung der Bildaufnahmeeinheit stets auf den Arbeitsbereich gerichtet bleibt.

In einer weiteren Ausgestaltung der Erfindung beinhalten die Positioniermittel eine Aktoreinheit zum Verschwenken der Bildaufnahmeeinheit sowie eine damit gekoppelte Sensoreinheit, mit der eine aktuelle Position des Arbeitsbereichs bestimmbar ist.

Diese Maßnahme kann alternativ zu einer mechanischen Zwangskopplung verwendet werden. Bevorzugt wird die Maßnahme jedoch in Ergänzung zu einer mechanischen Zwangskopplung verwendet, wobei die mechanische Zwangskopplung einerseits und die Sensor-/Aktoreinheit andererseits unterschiedliche Freiheitsgrade der Bildaufnahmeeinheit steuern. Die Maßnahme besitzt den Vorteil, daß eine Sensor-/Aktoreinheit eine elektronische Positionierung ermöglichen, was eine größere Flexibilität und einen größeren Gestaltungsspielraum zur Folge hat. Dies gilt sowohl für die Konstruktion der erfindungsgemäßen Vorrichtung als auch für ihre Verwendung im praktischen Einsatz.

In einer weiteren Ausgestaltung der zuvor genannten Maßnahme beinhaltet die Sensoreinheit Meßmittel zum Bestimmen einer relativen Position des Arbeitsinstruments bezogen auf den Führungsschaft.

Die Meßmittel können beispielsweise einen Strichcode, eine Widerstandsmessung, einen Winkeldecoder oder einen Positionssensor auf der Basis von Infrarot, Ultraschall oder elektromagnetischen Feldern beinhalten. Die Maßnahme besitzt den Vorteil, daß derartige Positionssensoren im Stand der Technik an sich hinreichend bekannt sind, so daß eine Positionsbestimmung hierdurch sehr einfach möglich ist. Die Bezugnahme auf den Führungsschaft ermöglicht darüber hinaus eine stets gleichbleibende und exakt bekannte Referenz.

In einer weiteren Ausgestaltung, die sowohl alternativ als auch ergänzend zu der zuvor genannten Maßnahme verwendet werden kann, beinhaltet die Sensoreinheit Bildverarbeitungsmittel zur Identifikation des distalen Endes des Arbeitsinstruments in dem aufgenommenen Abbild.

Diese Maßnahme besitzt den Vorteil, daß auf zusätzliche Meßmittel, beispielsweise in Form eines Positionssensors, verzichtet werden kann, wodurch der hierfür erforderliche Bauraum eingespart wird. In Ergänzung zu einem Positionssensor erhält man demgegenüber eine Redundanz, die eine Erhöhung der Zuverlässigkeit und Meßgenauigkeit ermöglicht.

Bevorzugt schließt die optische Achse in der Arbeitsposition einen Winkel von zumindest 10°, besonders bevorzugt einen Winkel zwischen 20° und 70°, mit der Längsmittelachse des Führungsschafts ein.

Ebenfalls bevorzugt befindet sich die Bildeintrittsöffnung der Bildaufnahmeeinheit in der Arbeitsposition in einem seitlichen Abstand von dem Führungsschaft, der größer ist als etwa 1 cm.

Aufgrund dieser Maßnahmen erhält der behandelnde Arzt einen optimalen Blickwinkel auf den Arbeitsbereich, was die Durchführung des Eingriffs wesentlich erleichtert. Ab einem Winkel von etwa 10° erhält der behandelnde Arzt einen hinreichenden seitlichen Blick (Perspektive) auf das distale Ende des Arbeitsinstruments. Optimal ist der Winkelbereich zwischen 20° und 70°. Die Maßnahme ist besonders vorteilhaft, wenn die Bildaufnahmeeinheit über dieselbe Einschnittsöffnung wie der Führungsschaft in den Körperhohlraum des Patienten eingeführt wird, da der behandelnde Arzt in diesem Fall ansonsten Nachteile im Bezug auf den Blickwinkel in Kauf nehmen müßte.

In einer weiteren Ausgestaltung der Erfindung ist die Bildaufnahmeeinheit eine integrierte Videosonde, die ein elektrisches Bildsignal von dem Arbeitsbereich bereitstellt.

Bevorzugt ist die Videosonde eine Stereo-Videosonde, die dem behandelnden Arzt ein nochmals besseres perspektivisch räumliches Bild ermöglicht, vor allem in Verbindung mit der Ausgestaltung nach Anspruch 3. Die Maßnahme besitzt allgemein den Vorteil, daß ein elektrisches Bildsignal, insbesondere in digitaler Form, ohne oder mit nur vergleichsweise geringen Qualitätsverlusten transportiert werden kann, weil keine Abbildungsfehler wie bei Linsensystemen auftreten. Infolgedessen ist die Bildwiedergabequalität hier besonders hoch. Gerade für Stereo-Bildaufnahmeeinheiten besitzt die Erfindung den zusätzlichen Vorteil, daß Doppelbilder und/oder Verzerrungen verringert sind, da ein stets gleichbleibender, optimaler Arbeitsabstand und somit eine konstante 3D-Perspektive erhalten bleiben.

Im Zusammenhang mit der zuvor genannten Maßnahme ist bevorzugt, wenn das von der Bildaufnahmeeinheit aufgenommene Bild telemetrisch übermittelt wird.

Hierbei ist von Vorteil, daß das von dem Videosensor aufgenommene Bild ohne aufwendige Kabelsysteme nach proximal übertragen werden kann. Im Zusammenhang mit dem zuvor erwähnten ein- oder mehrachsigen Gelenkmechanismus zum Ankoppeln der Bildaufnahmeeinheit an den Führungsschaft hat dies besonders Vorteile, da keine Kabel durch das oder die Gelenke des Schwenkmechanismus hindurchgeführt werden müssen. Auch die Reparaturanfälligkeit und die Dichtigkeit infolge der Verwendung von Kabeln ist durch die zuvor genannte Maßnahme wesentlich reduziert.

Dabei ist es weiterhin bevorzugt, wenn die Bildaufnahmeeinheit einen Sender aufweist, dessen gesendete Bildsignale von einem Empfänger empfangen werden.

Die Integration eines Senders in die Bildaufnahmeeinheit, d.h. in den Videosensor, hat den Vorteil, daß gänzlich auf Kabel zur Bildübertragung zwischen dem Videosensor und dem Empfänger verzichtet werden kann, so daß die Bildaufnahmeeinheit vollständig eingekapselt werden kann, wodurch Dichtigkeitsprobleme vollständig beseitigt werden können.

Dabei ist es weiterhin bevorzugt, wenn der Empfänger oder zumindest seine Antenne am intrakorporalen Abschnitt des Führungsschafts angeordnet ist.

Während es auch möglich ist, die telemetrische Übertragung von der Bildaufnahmeeinheit durch die Bauchdecke hindurch zu einem extrakorporal angeordneten Empfänger hin zu bewerkstelligen, hat die vorstehend genannte Maßnahme den Vorteil, daß auch höherfrequente Frequenzbereiche eingesetzt werden können, die ansonsten durch die Bauchdecke gedämpft würden.

In einer weiteren bevorzugten Ausgestaltung ist an der Bildaufnahmeeinheit eine Beleuchtungseinrichtung angeordnet, die zumindest eine Leuchtdiode aufweist.

Hierbei ist von Vorteil, daß auch für eine Lichtzuführung zum Arbeitsbereich auf Lichtleiter verzichtet werden kann, die nicht über den Gelenkmechanismus geführt werden könnten und somit durch den Führungsschaft geführt werden müßten. Eine Leuchtdiode an der Bildaufnahmeeinheit hat jedoch den wesentlichen Vorteil, daß die Richtung der Ausleuchtung und die Blickrichtung der Bildaufnahmeeinheit dieselbe ist, so daß bei einer Nachführung der Bildaufnahmeeinheit bei einer Bewegung des Arbeitsinstruments auch die Ausleuchtung optimal nachgeführt wird.

In einer weiteren bevorzugten Ausgestaltung weist die Bildaufnahmeeinheit eine Energiequelle, bspw. eine Batterie oder ein Akku zur Versorgung des Videosensors und ggf. der Leuchtdiode auf.

Insgesamt wird somit eine vollständig autonome Bildaufnahmeeinheit ggf. mit Lichtquelle zur Ausleuchtung des Arbeitsbereiches geschaffen, die im Zusammenhang mit der erfindungsgemäßen Positionierbarkeit der Bildaufnahmeeinheit vorteilhaft ist.

Aufbauend auf CMOS-Videosensoren ist es zukünftig denkbar, daß derartige autonome Bildaufnahmeeinheiten als Einwegprodukte hergestellt werden können, so daß die Reinigungs- und Wiederaufbereitungsproblematik nicht mehr existiert.

In einer alternativen Ausgestaltung der zuvor genannten Maßnahme ist die Bildaufnahmeeinheit ein optisches Element, das ein optisches Bildsignal von dem Arbeitsbereich bereitstellt.

Das optische Element kann beispielsweise ein geordnetes Faserbündel, ein Linsen- und/oder ein Spiegelsystem sein. Die Maßnahme besitzt den Vorteil, daß derartige passive Elemente sehr klein bauend und mit herkömmlichen, gut beherrschbaren Techniken realisiert werden können. Dies ist insbesondere dann vorteilhaft, wenn die Bildaufnahmeeinheit über dieselbe Öffnung wie der Führungsschaft in den Körperhohlraum des Patienten eingeführt werden soll.

In einer weiteren Ausgestaltung der Erfindung weist der Führungsschaft einen beidseitig offenen Führungskanal zum Aufnehmen und Führen von auswechselbaren Arbeitsinstrumenten auf.

Diese Maßnahme ermöglicht es dem behandelnden Arzt, verschiedene Arbeitsinstrumente in demselben Führungsschaft zu verwenden, wobei die Bildaufnahmeeinheit stets gleichbleibend auf den definierten Arbeitsbereich gerichtet bleiben kann. Infolgedessen kann sich der behandelnde Arzt auch beim Wechsel eines Arbeitsinstruments sehr schnell und einfach orientieren. Alternativ zu dieser Maßnahme wäre es jedoch auch möglich, die verschiedenen Arbeitsinstrumente jeweils mit einem eigenen Führungsschaft zu koppeln.

Wie bereits erwähnt, ist gemäß zuvor genannten Ausgestaltungen das Arbeitsinstrument in dem Führungsschaft in Axialrichtung beweglich geführt.

Diese Maßnahme besitzt den Vorteil, daß der behandelnde Arzt das Arbeitsinstrument in gewohnter Weise in dem Arbeitsbereich manipulieren kann und dabei beispielsweise Schnitte mit einer Schere in gewohnter Art und Weise ausführen kann.

In einer weiteren Ausgestaltung ist das Arbeitsinstrument in dem Führungsschaft in Radialrichtung fixiert.

Diese Maßnahme besitzt den Vorteil, daß der Führungsschaft radiale Bewegungen des Arbeitsinstruments unmittelbar nachvollzieht, was eine besonders einfache und effektive Zwangskopplung darstellt. Bei einer Rotation des Arbeitsinstruments im Führungsschaft wird der Führungsschaft vorzugsweise nicht mitbewegt, so daß die Blickrichtung der Bildaufnahmeeinheit dann unverändert bleibt, wie in einer weiteren bevorzugten Ausgestaltung vorgesehen ist.

Gegenüber einer vollständigen Radialfixierung kann es jedoch wiederum vorteilhaft sein, wenn das Arbeitsinstrument relativ zum Führungsschaft ein bestimmtes radiales Spiel aufweist, so daß bei einer seitlichen, quer zur Längsachse des Arbeitsinstruments erfolgenden Bewegung des Arbeitsinstruments die Bildaufnahmeeinheit innerhalb bestimmter Grenzen nicht nachgeführt und das Blickfeld unverändert bleibt, und erst bei über die Grenzen hinausgehender Bewegung eine Nachführung erfolgt, wie es für die axiale Beweglichkeit zuvor beschrieben wurde.

In einer weiteren Ausgestaltung der Erfindung weist die Bildaufnahmeeinheit Mittel zur Veränderung eines aufgenommenen Bildausschnitts auf.

Insbesondere besitzt die Bildaufnahmeeinheit dieser Ausgestaltung ein Zoomobjektiv, mit dem das Abbild des Arbeitsbereichs in vorgegebenen Grenzen vergrößert werden kann, ohne den räumlichen Abstand zwischen der Bildaufnahmeeinheit und dem Arbeitsbereich zu verändern. Hierdurch erhält der behandelnde Arzt eine weitere Möglichkeit, einen für die Durchführung der Behandlung optimalen Sichtbereich einzustellen, und zwar ohne dabei die Position des Arbeitsinstruments verändern zu müssen.

In einer weiteren Ausgestaltung der Erfindung ist an dem intrakorporalen Abschnitt des Führungsschaftes eine Beleuchtungseinrichtung angeordnet.

Auch diese Maßnahme besitzt den Vorteil, daß der Arbeitsbereich gut ausgeleuchtet ist. In Kombination mit der zuvor genannten Maßnahme ergeben sich zudem aufgrund der unterschiedlichen Beleuchtungsrichtungen unterschiedliche Schattenwürfe, die eine Erhöhung des Tiefeneindrucks bzw. des Stereoeffekts bewirken.

Ausführungsbeispiele der Erfindung sind in der Zeichnung dargestellt und werden in der nachfolgenden Beschreibung näher erläutert. Es zeigen:
- Fig. 1: ein erstes Ausführungsbeispiel einer erfindungsgemäßen Vorrichtung,
- Fig. 2: einen vergrößerten und teilweise geschnittenen Ausschnitt der Vorrichtung gemäß Fig. 1,
- Fig. 3: einen Führungsschaft mit einer Bildaufnahmeeinheit gemäß einem zweiten Ausführungsbeispiel der Erfindung,
- Fig. 4: den Führungsschaft aus Fig. 3 entlang der Linie IV-IV,
- Fig. 5: einen Führungsschaft mit einer Bildaufnahmeeinheit in Ruheposition gemäß einem dritten Ausführungsbeispiel der Erfindung,
- Fig. 6: den Führungsschaft aus Fig. 5, wobei die Bildaufnahmeeinheit in einer Arbeitsposition geschwenkt ist,
- Fig. 7: einen Führungsschaft sowei eine Bildaufnahmeeinheit gemäß einem vierten Ausführungsbeispiel der Erfindung,
- Fig. 8: ein weiteres Ausführungsbeispiel einer erfindungsgemäßen Vorrichtung,
- Fig. 9: eine Darstellung eines Arretiermechanismus zwischen einem Führungsschaft der Vorrichtung in Fig. 8 und einem darin geführten Arbeitsinstruments, und
- Fig. 10: ein gegenüber Fig. 9 abgewandeltes Ausführungsbeispiel eines Arretiermechanismus zwischen einem Führungsschaft der Vorrichtung in Fig. 8 und einem Arbeitsinstrument.

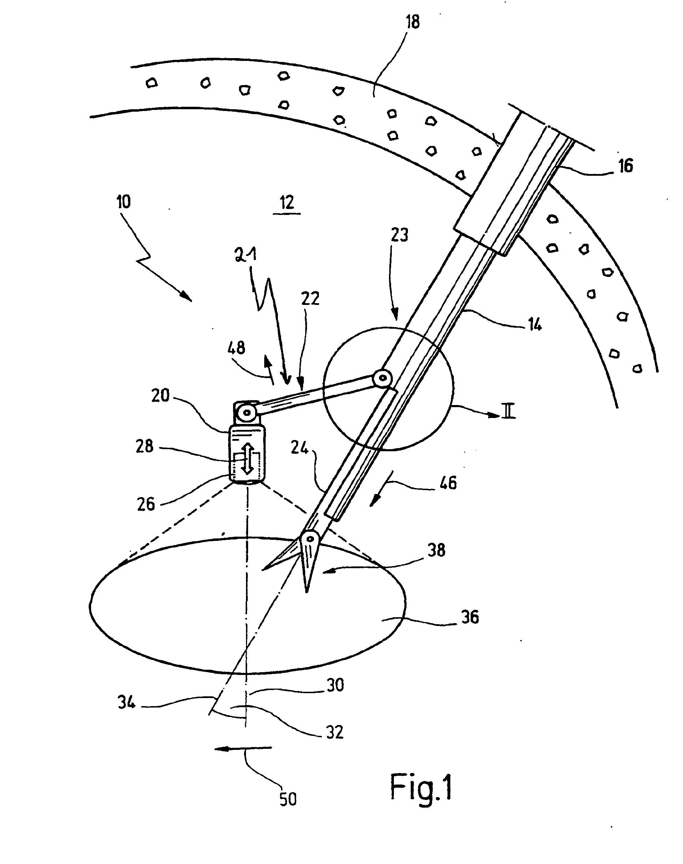

In Fig. 1 ist eine erfindungsgemäße Vorrichtung in ihrer Gesamtheit mit der Bezugsziffer 10 bezeichnet. Die Vorrichtung 10 dient zur Durchführung einer Operation im Bauchraum 12 eines Patienten.

Die Vorrichtung 10 beinhaltet einen Führungsschaft 14, der über einen Trokar 16 durch die Bauchdecke 18 des Patienten in den Bauchraum 12 eingeführt ist. Alternativ hierzu könnte der Führungsschaft 14 auch Bestandteil des Trokars 16 sein.

Mit der Bezugsziffer 20 ist eine Bildaufnahmeeinheit bezeichnet, die über eine Halterung 21, die einen mehrachsigen Gelenkmechanismus 22 aufweist, der im vorliegenden Fall zwei Gelenkachsen aufweist, an einem intrakorporalen Abschnitt 23 des Führungsschaftes 14 befestigt ist. Die Bildaufnahmeeinheit ist hier eine integrierte, miniaturisierte Videosonde, die über eine hier nicht dargestellte elektrische Verbindung ein elektrisches Bildsignal an eine außerhalb des Bauchraums 12 angeordnete Bildwiedergabeeinheit liefert.

Die Bildaufnahmeeinheit bzw. Videosonde 20 kann vorteilhafterweise auch einen nicht dargestellten Sender aufweisen, um das von dem Videosensor 20 aufgenommene Bild telemetrisch in Form von gesendeten Bildsignalen zu übermitteln. Zum Empfangen dieser Bildsignale ist dann ein nicht dargestellter Empfänger an dem intrakorporalen Abschnitt 23 des Führungsschafts 14 angeordnet.

Mit der Bezugsziffer 24 ist ein Arbeitsinstrument bezeichnet, das im vorliegenden Fall beispielhaft eine Faßzange ist. Allgemein kann das Arbeitsinstrument 24 ein beliebiges Instrument sein, das zur Durchführung des minimal-invasiven Eingriffs in dem Bauchraum 12 des Patienten benötigt wird.

Die Videosonde 20 besitzt im vorliegenden Ausführungsbeispiel ein Zoomobjektiv 26 mit variabler Brennweite, was durch einen Pfeil 28 angedeutet ist.

Mit der Bezugsziffer 30 ist die optische Achse der Videosonde 20 bezeichnet, die in der dargestellten Arbeitsposition unter einem Winkel 32 zu der Längsmittelachse 34 des Führungsschaftes 14 verläuft.

Mit der Bezugsziffer 36 ist der Arbeitsbereich des Arbeitsinstruments 24 bezeichnet, der im wesentlichen durch das distale Ende 38 des Arbeitsinstruments 24 definiert wird.

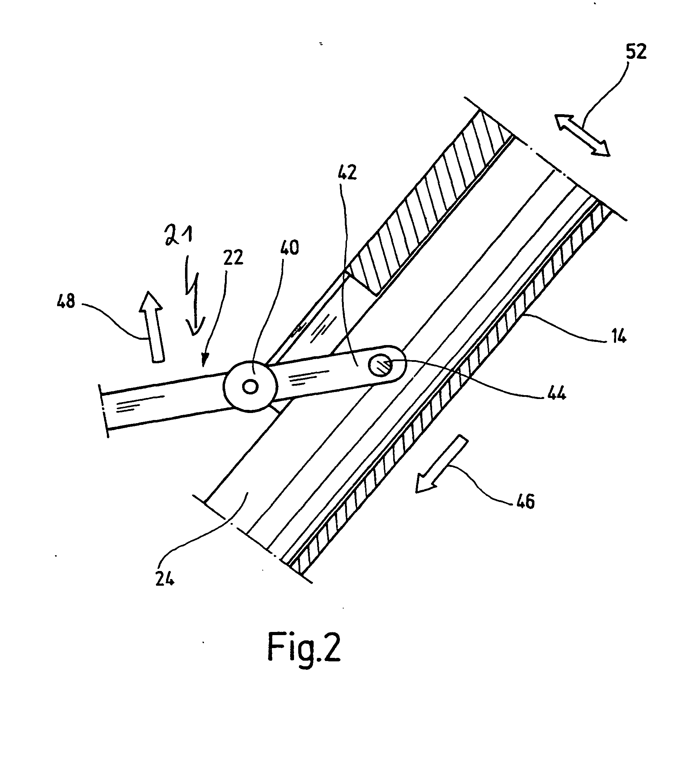

Die Videosonde 20 ist über den Gelenkmechanismus 22 sowie nachfolgend näher dargestellte Maßnahmen derart mit dem intrakorporalen Abschnitt 23 des Führungsschaftes 14 verbunden, daß ihre optische Achse 30 und damit ihr Blickwinkel räumlichen Veränderungen des Arbeitsbereichs 36 stets automatisch folgen kann. Zur Erläuterung dieser Funktion wird nachfolgend zusätzlich auf die Fig. 2 Bezug genommen. Gleiche Bezugszeichen bezeichnen dabei dieselben Elemente wie in Fig. 1.

Wie in Fig. 2 zu erkennen ist, ist der Gelenkmechanismus 22 über ein Gelenk 40 an dem Führungsschaft 14 befestigt, wobei das Gelenk 40 den Anlenkungspunkt der Halterung 21 am Führungsschaft 14 bildet. Von diesem Anlenkungspunkt ist die Bildaufnahmeeinheit 20 beabstandet an der Halterung 21, im gezeigten Ausführungsbeispiel am äußeren freien Ende angeordnet. Dabei weist der Gelenkmechanismus 22 einen Hebelarm 42 auf, der über das Gelenk 40 in das Innere des Führungsschaftes 14 hineinragt. An dem freien Ende des Hebelarms 42 ist eine federgelagerte Kugel 44 dargestellt, die hier beispielhaft für einen im übrigen nicht näher erläuterten Verriegelungsmechanismus steht.

Das Arbeitsinstrument 24 ist über die Kugel 44 bzw. über den hierdurch allgemeiner repräsentierten Verriegelungsmechanismus lösbar mit dem Hebelarm 42 verbunden. Diese Verbindung hat zur Folge, daß eine Bewegung des Arbeitsinstruments 24 in Richtung des Pfeils 46, d.h. eine Bewegung in Axialrichtung, den Gelenkmechanismus 22 in Richtung des Pfeils 48 kippt. Hierdurch verschiebt sich die optische Achse 30 der Videosonde 20 in Richtung des Pfeils 50, so daß der Blickwinkel der Videosonde 20 letztlich der axialen Bewegung 46 des Arbeitsinstruments 24 folgt.

Bei einer Bewegung des Arbeitsinstruments 24 entgegen der Richtung des Pfeils 46 verlagert sich der Blickwinkel der Videosonde 20 in umgekehrter Richtung, so daß die Videosonde 20 insgesamt aufgrund der mechanischen Zwangsführung über den Hebelarm 42 und die Kugel 44 einer Verschiebung des Arbeitsbereichs 36 automatisch nachgeführt wird.

In radialer Richtung, d.h. in Richtung des Pfeils 52 in Fig. 2 besitzt das Arbeitsinstrument 24 keinen Freiheitsgrad gegenüber dem Führungsschaft 14. Infolgedessen folgt der Führungsschaft 14 jeder Bewegung des Arbeitsinstruments 24 in Richtung des Pfeils 52. Wie leicht nachzuvollziehen ist, wird hierdurch die Blickrichtung der Videosonde 20 ebenfalls einer Verschiebung des Arbeitsbereichs 36 nachgeführt. Es kann jedoch ein gewisses radiales Spiel zwischen dem Arbeitsinstrument und dem Führungsschaft 14 vorgesehen sein, so daß die Nachführung nur bei Bewegungen des Arbeitsinstruments 24, die über das Spiel hinausgehen, erfolgt.

Eine automatische Nachführung der Bildaufnahmeeinheit 20 in Bezug auf axiale Bewegungen des Arbeitsinstruments 24 erübrigt sich, wenn das Blickfeld der Bildaufnahmeeinheit 20 so breit ist, daß das distale Ende 38 des Arbeitsinstruments 24 auch bei einer Manipulation durch den behandelnden Arzt stets sichtbar bleibt. Dies kann bei der Vorrichtung 10 beispielsweise dadurch berücksichtigt werden, daß das Arbeitsinstrument 24 gegenüber dem freien Ende des Hebelarms 42 ein Spiel besitzt, so daß der Hebelarm 42 Bewegungen des Arbeitsinstruments 24 erst ab einer vorgegebenen Stärke folgt.

Bei der nachfolgenden Beschreibung der weiteren Ausführungsbeispiele bezeichnen gleiche Bezugszeichen weiterhin dieselben Elemente wie in den vorhergehenden Figuren.

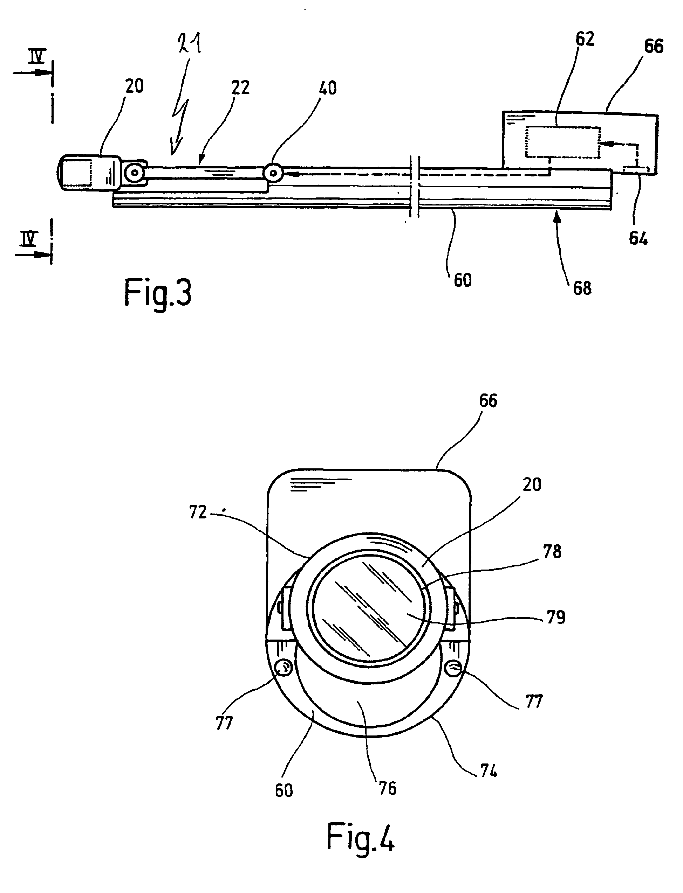

Der Führungsschaft 60 in Fig. 3 unterscheidet sich von dem Führungsschaft 14 des ersten Ausführungsbeispiels im wesentlichen durch eine Aktoreinheit 62 sowie eine damit verbundene Sensoreinheit 64, die in einem gemeinsamen Gehäuse 66 am proximalen Ende 68 des Führungsschaftes 60 angeordnet sind. Die Aktoreinheit 62 beinhaltet eine hier nicht näher dargestellte Auswerteeinheit sowie einen ebenfalls nicht näher dargestellten Stellantrieb, der die den Gelenkmechanismus 22 aufweisende Halterung 21 und damit die Ausrichtung der Videosonde 20 ansteuert. Als Stellantrieb wird bevorzugt ein Schrittmotor verwendet.

Die Sensoreinheit beinhaltet in diesem Ausführungsbeispiel einen Positionssensor, der die relative Position eines in dem Führungsschaft 60 geführten Arbeitsinstruments 24 (hier nicht dargestellt) bezogen auf den Führungsschaft 60 bestimmt. Aus der erhaltenen Position läßt sich auf die aktuelle Position des Arbeitsbereichs 36 schließen, so daß die Aktoreinheit 62 die Videosonde 20 entsprechend nachführen kann.

In Fig. 3 ist die Videosonde 20 an dem Führungsschaft 60 in eine Ruheposition geschwenkt, die es möglich macht, den Führungsschaft 60 mitsamt der Videosonde 20 durch den Trokar 16 in den Bauchraum 12 des zu behandelnden Patienten einzuführen. Wie in der Frontalansicht gemäß Fig. 4 zu erkennen ist, befindet sich die Videosonde 20 dabei in einer solchen Position, daß ihre äußere Querschnittskontur 72 im wesentlichen innerhalb von und damit deckungsgleich zu der äußeren Querschnittskontur des Führungsschafts 60 angeordnet ist. Die Arbeitsposition der Videosonde 20 des Führungsschafts 60 entspricht der Darstellung des Führungsschafts 14 in Fig. 1.

In der Darstellung in Fig. 4 ist des weiteren ein Führungskanal 76 des Führungsschafts 60 erkennbar, der beidseitig, d.h. sowohl am distalen Ende 38 als auch am proximalen Ende 68, offen ist. Hierdurch ist es möglich, im Verlauf der Operation verschiedene Arbeitsinstrumente 24 in den Führungsschaft 60 einzuführen bzw. aus diesem zu entnehmen.

Mit den Bezugsziffern 77 und 78 sind zwei Beleuchtungseinrichtungen bezeichnet, die an dem distalen Ende des Führungsschaftes 60 und an der Bildaufnahmeeinheit 20 angeordnet sind. Die Beleuchtungseinrichtung 77 beinhaltet dabei zwei LED's, die in den Führungsschaft 60 zu beiden Seiten des Führungskanals 76 integriert sind. Die Beleuchtungseinrichtung 78 beinhaltet demgegenüber ein ungeordnetes Faserbündel, wobei die Faserenden konzentrisch zu der Bildeinlaßöffnung 79 angeordnet sind. Anstelle der Faserbündel können jedoch ebenfalls LED's an der Bildaufnahmeeinheit 20 angeordnet sein. Im in Zusammenhang mit Fig. 1 beschriebener Ausgestaltung der Bildaufnahmeeinheit kann zur Ansteuerung und Versorgung der Videosonde und der zuvor genannten LED's in der Bildaufnahmeeinheit des weiteren eine Energiequelle, insbesondere eine Batterie oder ein Akku, vorgesehen sein, so daß die Bildaufnahmeeinheit insgesamt autonom arbeitet.

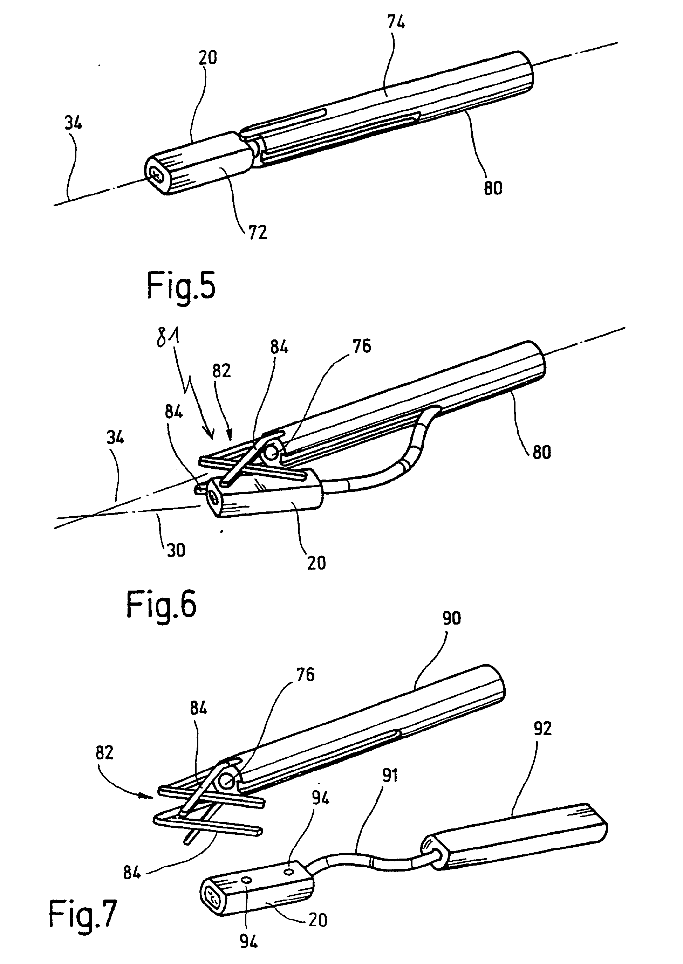

In den Figuren 5 und 6 ist ein weiteres Ausführungsbeispiel eines erfindungsgemäßen Führungsschafts mit der Bezugsziffer 80 bezeichnet. Der Führungsschaft 80 ist über eine einen mehrachsigen Gelenkmechanismus 82 aufweisende Halterung 81 mit einer Videosonde 20 verbunden. In seinem Inneren weist der Führungsschaft 80 einen beidseitig offenen Führungskanal 76 zum Aufnehmen und Führen von auswechselbaren Arbeitsinstrumenten 24 auf.

Im Unterschied zu den vorhergehenden Ausführungsbeispielen besitzt der Gelenkmechanismus 82 hier zwei parallel zueinander angeordnete Scherenglieder 84, zwischen denen die Videosonde 20 verschwenkbar gehalten ist. Wie aus der Darstellung in Fig. 5 erkennbar ist, kann die Videosonde 20 dabei ebenfalls in eine Ruheposition geschwenkt werden, bei der ihre äußere Querschnittskontur 72 innerhalb der äußeren Querschnittskontur 74 des Führungsschafts 80 angeordnet ist. In diesem Fall ist die äußere Querschnittskontur 72 der Videosonde 20 vollkommen dekkungsgleich mit der äußeren Querschnittskontur 74 des Führungsschaftes 80.

Die Funktionsweise des Führungsschaftes 80 entspricht derjenigen der vorhergehenden Ausführungsbeispiele, wobei der Führungsschaft 80 alternativ oder ergänzend zueinander sowohl mit einer mechanischen Zwangskopplung als auch mit einer Sensor-/Aktoreinheit zum Nachführen der Videosonde versehen sein kann.

In Fig. 7 ist als weiteres Ausführungsbeispiel der Erfindung ein Führungsschaft 90 dargestellt, der weitgehend dem Führungsschaft 80 gemäß den Figuren 5 und 6 entspricht. Im Unterschied dazu kann die Videosonde 20 hier jedoch von dem Gelenkmechanismus 82 abgetrennt und intrakorporal angekoppelt werden. Hierdurch ist es möglich, die Videosonde 20 und den Führungsschaft 90 über verschiedene Einschnitte in den Bauchraum 12 eines Patienten einzubringen. Die Videosonde 20 ist dabei über ein Kabel 91 mit einem eigenen Schaft 92 verbunden. Die Kopplung der Videosonde 20 mit dem Gelenkmechanismus 82 erfolgt hier bevorzugt mit Hilfe von Elektromagneten 94, die an der Außenseite der Videosonde 20 angeordnet sind. Die Nachführung der Videosonde 20 erfolgt bei diesem Ausführungsbeispiel auf die bereits zuvor beschriebene Weise.

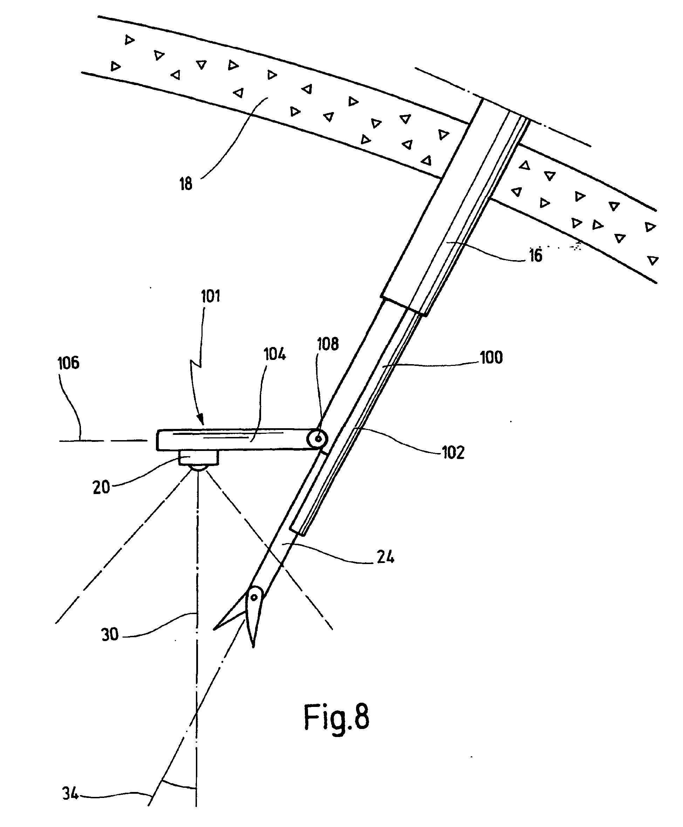

In Fig. 8 bis 10 ist als weiteres Ausführungsbeispiel der Erfindung ein Führungsschaft 100 dargestellt, der dem Führungsschaft 14 gemäß Fig. 1 und 2 ähnlich ist. Jedoch unterscheidet sich das Ausführungsbeispiel gemäß Fig. 8 bis 10 durch die Ankopplung der Bildaufnahmeeinheit 20 an dem Führungsschaft 100 und der Art der Nachführung der Bildaufnahmeeinheit 20 bezüglich des Arbeitsinstruments 24.

Die Bildaufnahmeeinheit 20 ist an dem Führungsschaft 100 über eine Halterung 101 befestigt, die einen gelenkig am intrakorporalen Abschnitt 102 des Führungsschafts 100 befestigten Schwenkarm 104 aufweist. Der Schwenkarm 104 bildet somit einen einachsigen Gelenkmechanismus zur Verbindung der Bildaufnahmeeinheit 20 mit dem Führungsschaft 100.

Die relativ zum Schwenkarm 104 unbewegliche Bildaufnahmeeinheit 20 ist am freien Ende des zumindest einen Schwenkarms 104 so angeordnet, daß die optische Achse 30 etwa senkrecht zur Längsachse 106 des Schwenkarms 104 verläuft und dabei zur Längsmittelachse 34 des Führungsschafts 100 bzw. des Arbeitsinstruments 24 hinweist.

Der Schwenkarm 104 ist über ein Gelenk 108 verschwenkbar mit dem Führungsschaft 100 verbunden, wobei die Verschwenkbarkeit des Schwenkarms 104 wie bei den zuvor im Zusammenhang mit den anderen Ausführungsbeispielen beschriebenen Halterungen derart ist, daß die optische Achse 30 mit der Längsmittelachse 34 des Führungsschafts 100 einen Winkel von zumindest 10°, bevorzugt zwischen 20° und 70° einschließen kann, wenn die Vorrichtung in den Körperhohlraum eingeführt ist.

In den Arbeitspositionen ist die Bildaufnahmeeinheit 20 mehr als etwa 1 cm vom Führungsschaft 100 seitlich beabstandet, wozu der Schwenkarm 104 eine entsprechende Länge aufweist.

Der Schwenkarm 104 kann des weiteren teleskopartig ausgebildet sein, so daß die Länge des Schwenkarms 104 und damit der Abstand der Bildaufnahmeeinheit 20 vom Anlenkungspunkt am Führungsschaft 100, der durch das Gelenk 108 gebildet wird, vergrößerten werden kann, um den zuvor genannten Winkelbereich und den seitlichen Abstand einhalten zu können.

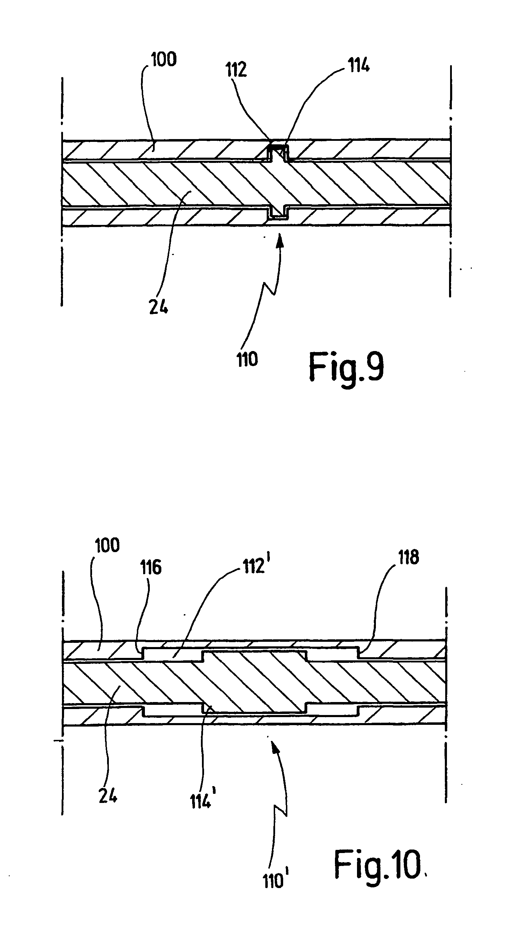

Während für die Halterung 101 und damit die Bildaufnahmeeinheit 20 ein mit Fig. 2 vergleichbarer Mechanismus zum Nachführen der optischen Achse 30, d.h. der Blickrichtung der Bildaufnahmeeinheit 20 an eine Bewegung des Arbeitsinstruments 24, insbesondere einer Axialverschiebung des Arbeitsinstruments 24 auch bei dem Ausführungsbeispiel in Fig. 8 vorgesehen werden kann, wird mit Bezug auf Fig. 9 und 10 nun ein besonders einfacher Nachführmechanismus beschrieben.

Anstatt daß das Arbeitsinstrument 24 wie bei dem in Fig. 2 gezeigten Ausführungsbeispiel relativ zum Führungsschaft 100 axial frei verschiebbar ist, und die Halterung Koppelmittel in Form des Hebels 42 und der Kugel 44 aufweist, die mit dem Arbeitsinstrument 24 in Eingriff bringbar sind, so daß bei einer Relativverschiebung zwischen Arbeitsinstrument 24 und Führungsschaft 100 die Halterung 101 verschwenkt wird, um die optische Achse 30 dem Arbeitsbereich 36 nachzuführen, sind für den Führungsschaft 100 Arretiermittel 110 vorgesehen, um das Arbeitsinstrument 24 zumindest teilweise axial relativ zum Führungsschaft 100 festzulegen.

Die Arretiermittel 110 weisen gemäß Fig. 9 eine am Führungsschaft 100, vorzugsweise in seinem extrakorporalen Abschnitt, eine Ringnut 112 auf, in der ein am Arbeitsinstrument 24 vorgesehener Zapfen 114 läuft. Der Zapfen 114 ist vorzugsweise gefedert, so daß er durch einen entsprechenden nicht dargestellten Rastmechanismus mit der Ringnut 112 außer Eingriff gebracht werden kann und selbsttätig in die Ringnut 112 einrasten kann. Das Arbeitsinstrument 24 bleibt durch die Arretiermittel 110 in dem Führungsschaft 100 um seine Längsachse frei drehbar.

Während sich bei einer Drehung des Arbeitsinstruments 24 um seine Längsachse der Führungsschaft 100 somit nicht mitdreht, und sich die eingestellte Blickrichtung der Bildaufnahmeeinheit 20 somit nicht ändert, wird bei einer axialen Verschiebung des Arbeitsinstruments 24 der Führungsschaft 100 in der gleichen Richtung mitbewegt, und über die mechanische Ankopplung der Bildaufnahmeeinheit 20 über den Schwenkarm 104 an dem Führungsschaft 100 wird diese in der gleichen Weise parallel verschoben. Bei einer Drehung des Arbeitsinstruments 24 um seine Längsachse bleibt die Bildaufnahmeeinheit 20 unverändert in ihrer Position.

Die Arretiermittel 110 bewirken demnach ein vollständiges axiales Festlegen des Arbeitsinstruments 24 relativ zum Führungsschaft 100.

Demgegenüber ist in Fig. 10 ein abgewandeltes Ausführungsbeispiel von Arretiermitteln 110' dargestellt, die so ausgebildet sind, daß das Arbeitsinstrument 24 innerhalb vorbestimmter Grenzen axial relativ zum Führungsschaft 100 verschiebbar ist, bei einer über die vorbestimmten axialen Grenzen hinausgehenden Verschiebung den Führungsschaft 100 jedoch axial mitnimmt.

Dazu sind die Arretiermittel 110' in mit Fig. 9 vergleichbarer Weise mit einer Ringnut 112' am Führungsschaft 100 und einem Zapfen 114' am Arbeitsinstrument 24 ausgebildet, wobei der Zapfen 114' axial kürzer ist als die Ringnut 112'.

Das Arbeitsinstrument 24 läßt sich somit relativ zum Führungsschaft 100 axial frei über eine Strecke verschieben, die der Differenz der axialen Länge der Ringnut 112' und der axialen Länge des Zapfens 114' entspricht. Die vorgenannten Grenzen der axialen freien Beweglichkeit werden somit durch das vordere Ende 116 und das hintere Ende 118 der Ringnut 112' und durch die axiale Länge des Zapfens 114' bestimmt.

Innerhalb dieser freien axialen Beweglichkeit des Arbeitsinstruments 24 relativ zu dem Führungsschaft 100 wird bei einer Verschiebung des Arbeitsinstruments 24 demnach der Führungsschaft 100 nicht mit verschoben, wodurch entsprechend die Bildaufnahmeeinheit 20 ebenfalls nicht bewegt wird und somit die Blickrichtung der optischen Achse 30 erhalten bleibt. Die Spitze des Arbeitsinstruments 24 kann demnach innerhalb der vorbestimmten Grenzen im unveränderten Bildfeld bewegt werden. Die zuvor genannten Grenzen der relativen Verschiebbarkeit zwischen den Arbeitsinstrument 24 und dem Führungsschaft 100 werden vorteilhafterweise so eingestellt, daß der Bereich der freien axialen Beweglichkeit gerade der Strecke zwischen dem Ein- und Austreten der Spitze des Arbeitsinstruments 24 aus dem Bildfeld entspricht. Erst wenn die Spitze des Arbeitsinstruments 24 aus dem Bildfeld herauswandern würde, wird der Führungsschaft 100 und damit das Bildaufnahmesystem 20 mit bewegt.

## Patentansprüche

1. Vorrichtung zur intrakorporalen, minimal-invasiven Behandlung eines Patienten, mit einem Arbeitsinstrument (24), das zur Durchführung eines Behandlungsschrittes in einen Körperhohlraum (12) des Patienten einführbar ist, wobei ein distales Ende (38) des eingeführten Arbeitsinstruments (24) einen intrakorporalen Arbeitsbereich (36) definiert, und mit zumindest einer Bildaufnahmeeinheit (20) zum Aufnehmen eines Abbildes des intrakorporalen Arbeitsbereichs (36), ferner mit Positioniermitteln zum Ausrichten einer optischen Achse (30) der Bildaufnahmeeinheit (20) in Abhängigkeit von einer räumlichen Lage des intrakorporalen Arbeitsbereichs (36), wobei die Positioniermittel einen Führungsschaft (14; 60; 80; 90; 100) beinhalten, in dem das Arbeitsinstrument (24) geführt ist, und wobei die Bildaufnahmeeinheit (20) verschwenkbar an einem intrakorporalen Abschnitt (23) des Führungsschaftes (22, 42, 44; 22, 62, 64) befestigt ist, **dadurch gekennzeichnet, daß** die Positioniermittel eine am intrakorporalen Abschnitt (23) des Führungsschafts (14; 60; 80; 90; 100) verschwenkbar befestigte Halterung (21; 81; 101) aufweisen, an der die Bildaufnahmeeinheit (20) von einer Anlenkstelle der Halterung (21; 81; 101) am Führungsschaft (14; 60; 80; 100) beabstandet angeordnet ist, derart, daß die Bildaufnahmeeinheit (20) intrakorporal in eine Arbeitsposition schwenkbar ist, in der die optische Achse (30) zu einer Längsmittelachse (34) des Führungsschaftes (14; 80) hinweisend winklig zu dieser verläuft.

2. Vorrichtung nach Anspruch 1, **dadurch gekennzeichnet, daß** die Halterung zumindest einen gelenkig mit dem intrakorporalen Abschnitt befestigten Schwenkarm (22; 84; 104) aufweist.

3. Vorrichtung nach Anspruch 2, **dadurch gekennzeichnet, daß** die Bildaufnahmeeinheit (20) am freien Ende des zumindest einen Schwenkarms (104) so angeordnet ist, daß die optische Achse (30) der Bildaufnahmeeinheit (20) etwa senkrecht zur Längsachse (106) des Schwenkarms (104) verläuft und zur Längsmittelachse (34) des Führungsschafts (100) hin weist.

4. Vorrichtung nach Anspruch 3, **dadurch gekennzeichnet, daß** der Schwenkarm (104) eine verstellbare Länge aufweist.

5. Vorrichtung nach einem der Ansprüche 1 bis 4, **dadurch gekennzeichnet, daß** die Halterung einen mehrachsigen Gelenkmechanismus (22; 82) aufweist.

6. Vorrichtung nach einem der Ansprüche 1 bis 5, **dadurch gekennzeichnet, daß** die Bildaufnahmeeinheit (20) in eine Ruheposition an dem Führungsschaft (60; 80) schwenkbar ist, in der eine äußere Querschnittskontur (72) der Bildaufnahmeeinheit (20) im wesentlichen deckungsgleich mit einer äußeren Querschnittskontur (74) des Führungsschaftes (60; 80) angeordnet ist.

7. Vorrichtung nach einem der Ansprüche 1 bis 6, **dadurch gekennzeichnet, daß** die Bildaufnahmeeinheit (20) über einen intrakorporal aktivierbaren Koppelmechanismus (94) an dem Führungsschaft (90) fixierbar ist.

8. Vorrichtung nach einem der Ansprüche 1 bis 7, **dadurch gekennzeichnet, daß** die Positioniermittel eine mechanische Zwangskopplung zwischen dem Arbeitsinstrument (24) und der Bildaufnahmeeinheit (20) beinhalten.

9. Vorrichtung nach Anspruch 8, **dadurch gekennzeichnet, daß** die Positioniermittel Arretiermittel (110) zum zumindest teilweisen axialen Festlegen des Arbeitsinstruments (24) relativ zum Führungsschaft (100) aufweisen.

10. Vorrichtung nach Anspruch 9, **dadurch gekennzeichnet, daß** die Arretiermittel (110) so ausgebildet sind, daß das Arbeitsinstrument (24) innerhalb vorbestimmter Grenzen (116, 118) axial relativ zum Führungsschaft (110) frei verschiebbar ist, bei einer über die vorbestimmten axialen Grenzen (116, 118) hinausgehenden Verschiebung den Führungsschaft (100) jedoch axial mitnimmt.

11. Vorrichtung nach Anspruch 8, **dadurch gekennzeichnet, daß** das Arbeitsinstrument (24) relativ zum Führungsschaft (14) axial frei verschiebbar ist, und daß die Halterung (21) Koppelmittel (42, 44) aufweist, die mit dem Arbeitsinstrument (24) in Eingriff bringbar sind, derart, daß bei einer Relativverschiebung zwischen Arbeitsinstrument (24) und Führungsschaft (14) die Halterung (21) verschwenkt wird, um die optische Achse (30) dem Arbeitsbereich (36) nachzuführen.

12. Vorrichtung nach einem der Ansprüche 1 bis 11, **dadurch gekennzeichnet, daß** die Positioniermittel eine Aktoreinheit (62) zum Verschwenken der Bildaufnahmeeinheit (20) sowie eine damit gekoppelte Sensoreinheit (64) beinhalten, mit der eine aktuelle Position des Arbeitsbereichs (36) bestimmbar ist.

13. Vorrichtung nach Anspruch 12, **dadurch gekennzeichnet, daß** die Sensoreinheit (62) Meßmittel zum Bestimmen einer relativen Position des Arbeitsinstruments (24) bezogen auf den Führungsschaft (60) beinhaltet.

14. Vorrichtung nach Anspruch 12 oder 13, **dadurch gekennzeichnet, daß** die Sensoreinheit Bildverarbeitungsmittel zur Identifikation des distalen Endes des Arbeitsinstruments in dem aufgenommenen Abbild beinhaltet.

15. Vorrichtung nach einem der Ansprüche 1 bis 14, **dadurch gekennzeichnet, daß** die optische Achse (30) in der Arbeitsposition einen Winkel (32) von zumindest 10°, bevorzugt zwischen 20° und 70°, mit der Längsmittelachse (34) des Führungsschaftes (14) einschließt.

16. Vorrichtung nach einem der Ansprüche 1 bis 15, **dadurch gekennzeichnet, daß** sich eine Bildeintrittsöffnung (79) der Bildaufnahmeeinheit (20) in der Arbeitsposition in einem seitlichen Abstand von dem Führungsschaft (14; 60; 80; 90; 100), der größer ist als etwa 1 cm, befindet.

17. Vorrichtung nach einem der Ansprüche 1 bis 15, **dadurch gekennzeichnet, daß** die Bildaufnahmeeinheit (20) eine integrierte Videosonde ist, die ein elektrisches Bildsignal von dem Arbeitsbereich (36) bereitstellt.

18. Vorrichtung nach Anspruch 17, **dadurch gekennzeichnet, daß** das von der Bildaufnahmeeinheit (20) aufgenommene Bild telemetrisch übermittelt wird.

19. Vorrichtung nach Anspruch 18, **dadurch gekennzeichnet, daß** die Bildaufnahmeeinheit (20) einen Sender aufweist, dessen gesendete Bildsignale von einem Empfänger empfangen werden.

20. Vorrichtung nach Anspruch 19, **dadurch gekennzeichnet, daß** der Empfänger oder zumindest seine Antenne am intrakorporalen Abschnitt (23) des Führungsschafts (14; 60; 80; 90; 100) angeordnet ist.

21. Vorrichtung nach einem der Ansprüche 18 bis 20, **dadurch gekennzeichnet, daß** an der Bildaufnahmeeinheit eine Beleuchtungseinrichtung (77) angeordnet ist, die vorzugsweise zumindest eine Leuchtdiode aufweist.

22. Vorrichtung nach einem der Ansprüche 18 bis 21, **dadurch gekennzeichnet, daß** die Bildaufnahmeeinheit eine Energiequelle, bspw. eine Batterie oder ein Akku zu ihrer Versorgung aufweist.

23. Vorrichtung nach einem der Ansprüche 1 bis 22, **dadurch gekennzeichnet, daß** die Bildaufnahmeeinheit ein optisches Element ist, das ein optisches Bildsignal von dem Arbeitsbereich bereitstellt.

24. Vorrichtung nach einem der Ansprüche 1 bis 23, **dadurch gekennzeichnet, daß** der Führungsschaft (14; 60; 80; 90) einen beidseitig offenen Führungskanal (76) zum Aufnehmen und Führen von auswechselbaren Arbeitsinstrumenten (24) aufweist.

25. Vorrichtung nach einem der Ansprüche 1 bis 24, **dadurch gekennzeichnet, daß** das Arbeitsinstrument (24) in dem Führungsschaft in Radialrichtung (52) fixiert ist.

26. Vorrichtung nach einem der Ansprüche 1 bis 19, **dadurch gekennzeichnet, daß** das Arbeitsinstrument (24) im Führungsschaft (14; 60; 80; 90; 100) um seine Längsachse frei drehbar ist.

27. Vorrichtung nach einem der Ansprüche 1 bis 26, **dadurch gekennzeichnet, daß** die Bildaufnahmeeinheit (20) Mittel (26) zur Veränderung eines aufgenommenen Bildausschnitts aufweist.

28. Vorrichtung nach einem der Ansprüche 1 bis 27, **dadurch gekennzeichnet, daß** an dem intrakorporalen Abschnitt (23) des Führungsschaftes (60) eine Beleuchtungseinrichtung (77) angeordnet ist.

29. Führungsschaft zur Verwendung in einer Vorrichtung zur intrakorporalen, minimal-invasiven Behandlung eines Patienten; wobei in die Vorrichtung ein Arbeitsinstrument, (24) zur Durchführung eines Behandlungsschrittes in einem Körperhohlraum einführbar ist, und die Vorrichtung zumindest eine eine optische Achse (30) aufweisende Bildaufnahmeeinheit (20) aufweist, wobei der Führungsschaft (14; 60; 80; 90; 100) einen intrakorporalen Abschnitt (23) aufweist, der Positioniermittel zur verschwenkbaren Befestigung der Bildaufnahmeeinheit (20) aufweist, **dadurch gekennzeichnet, dass** die Positioniermittel eine am intrakorporalen Abschnitt (23) des Führungsschafts (14; 60; 80; 90; 100) verschwenkbar befestigte Halterung (21; 81; 101) aufweisen, an der die Bildaufnahmeeinheit (20) von einer Anlenkstelle der Halterung (21; 81; 101) am Führungsschaft (14; 60; 80; 90; 100) beabstandet angeordnet ist, derart, dass die Bildaufnahmeeinheit (20) intrakorporal in eine Arbeitsposition schwenkbar ist, in der die optische Achse (30) zu einer Längsmittelachse (34) des Führungsschaftes (14; 60; 80; 90; 100) hinweisend winklig zu dieser verläuft.

## Claims

1. A device for intracorporal, minimal-invasive treatment of a patient, comprising a working instrument (24) that can be introduced into a body cavity (12) of the patient for carrying out a treatment step, wherein a distal end (38) of the introduced working instrument (24) defines an intracorporal working area (36), and at least one image pick-up unit (20) for picking up an image of the intracorporal working area (36), further comprising positioning means for orienting an optical axis (30) of the image pick-up unit (20) in dependency on a spatial position of the intracorporal working area (36), wherein the positioning means comprise a guide shaft (14; 60; 80; 90; 100), in which the working instrument (24) is guided, and wherein the image pick-up unit (20) is pivotably fixed at an intracorporal portion (23) of the guide shaft (22, 42, 44; 22, 62, 64), **characterized in that** the positioning means comprises a holder (21; 81; 101) pivotably fixed to the intracorporal portion (23) of the guide shaft (14; 60; 80; 90; 100), the image pick-up unit (20) being arranged at the holder (21; 81; 101) in a distance from a location where the holder (21; 81; 101) is linked to the guide shaft (14; 60; 80; 100), such that the image pick-up unit (20) is intracorporally pivotable into a working position, in which the optical axis (30) runs angularly to a longitudinal center axis (34) of the guide shaft (14; 60; 80; 90; 100) and points to the longitudinal center axis (34).

2. The device of claim 1, **characterized in that** the holder has at least one pivot arm (22; 84; 104) that is articulatedly fixed to the intracorporal portion.

3. The device of claim 2, **characterized in that** the image pick-up unit (20) is arranged at the free end of the at least one pivot arm (104) in such a way that the optical axis (30) of the image pick-up unit (20) runs approximately perpendicular to the longitudinal axis (106) of the pivot arm (104) and points to the longitudinal center axis (34) of the guide shaft (100).

4. The device of claim 3, **characterized in that** the pivot arm (104) has an adjustable length.

5. The device of anyone of claims 1 through 4, **characterized in that** the holder has a multi-axis articulation mechanism (22; 82).

6. The device of anyone of claims 1 through 5, **characterized in that** the image pick-up unit (20) is pivotable into a resting position at the guide shaft (60; 80), in which an outer cross-sectional contour (72) of the image pick-up unit (20) is arranged in an essentially congruent manner with respect to an outer cross-sectional contour (74) of the guide shaft (60; 80).

7. The device of anyone of claims 1 through 6, **characterized in that** the image pick-up unit (20) is fixable via an intracorporally activatable coupling mechanism (94) at the guide shaft (90).

8. The device of anyone of claims 1 through 7, **characterized in that** the positioning means comprise a mechanically constrained coupling between the working instrument (24) and the image pick-up unit (20).

9. The device of claim 8, **characterized in that** the positioning means have locking means (110) for an at least partial axial immobilization of the working instrument (24) with respect to the guide shaft (100).

10. The device of claim 9, **characterized in that** the locking means (110) are configured in such a way that the working instrument (24) is axially freely displaceable with respect to the guide shaft (110) within predetermined limits (116, 118), but axially entrains the guide shaft (100), if the working instrument is displaced beyond the predetermined axial limits (116, 118).

11. The device of claim 8, **characterized in that** the working instrument (24) is axially freely displaceable with respect to the guide shaft (14), and that the holder (21) has coupling means (42, 44), which can be brought in engagement with the working instrument (24) in such a way that, when the working instrument (24) is displaced relative to the guide shaft (14), the holder (21) is pivoted, in order to track the optical axis (30) onto the working area (36).

12. The device of anyone of claims 1 through 11, **characterized in that** the positioning means comprise an actuator unit (62) for pivoting the image pick-up unit (20) and a sensor unit (64) coupled therewith, by which a current position of the working area (36) can be determined.

13. The device of claim 12, **characterized in that** the sensor unit (62) comprises measuring means for determining a relative position of the working instrument (24) with reference to the guide shaft (60).

14. The device of claim 12 or 13, **characterized in that** the sensor unit comprises image processing means for identification of the distal end of the working instrument in the image picked up.

15. The device of anyone of claims 1 through 14, **characterized in that**, in the working position, the optical axis (30) encloses an angle (32) of at least 10°, preferably between 20° and 70°, with the longitudinal center axis (34) of the guide shaft (14).

16. The device of anyone of claims 1 through 15, **characterized in that**, in the working position, an image entrance opening (79) of the image pick-up unit (20) is in a lateral distance from the guide shaft (14; 60; 80; 90; 100), which is larger than approximately 1 cm.

17. The device of anyone of claims 1 through 15, **characterized in that** the image pick-up unit (20) is an integrated video probe, which provides an electrical image signal of the working area (36).

18. The device of claim 17, **characterized in that** the image picked up by the image pick-up unit (20) is telemetrically transmitted.

19. The device of claim 18, **characterized in that** the image pick-up unit (20) has a transmitter, the transmitted image signals of which are received by a receiver.

20. The device of claim 19, **characterized in that** the receiver or at least its antenna is arranged at the intracorporal portion (23) of the guide shaft (14; 60; 80; 90; 100).

21. The device of anyone of claims 18 through 20, **characterized in that** an illuminating device (77) is arranged at the image pick-up unit, which has preferably at least one light emitting diode.

22. The device of anyone of claims 18 through 21, **characterized in that** the image pick-up unit has a source of energy, e.g. a battery or an accumulator, for its supply.

23. The device of anyone of claims 1 through 22, **characterized in that** the image pick-up unit is an optical element, which provides an optical image signal of the working area.

24. The device of anyone of claims 1 through 23, **characterized in that** the guide shaft (14; 60; 80; 90) has a guide channel (76) that is open on both ends for receiving and guiding exchangeable working instruments (24).

25. The device of anyone of claims 1 through 24, **characterized in that** the working instrument (24) is immovable in radial direction (52) in the guide shaft.

26. The device of anyone of claims 1 through 19, **characterized in that** the working instrument (24) is freely rotatable about its longitudinal axis in the guide shaft (14; 60; 80; 90; 100).

27. The device of anyone of claims 1 through 26, **characterized in that** the image pick-up unit (20) has means (26) for modification of a picked up image sector.

28. The device of anyone of claims 1 through 27, **characterized in that** an illuminating device (77) is arranged on the intracorporal portion (23) of the guide shaft (60).

29. A guide shaft for use in a device for intracorporal, minimal-invasive treatment of a patient, wherein a working instrument (24) can be introduced into the device for carrying out a treatment step in a body cavity, and the device comprises at least one image pick-up unit (20) having an optical axis (30), wherein the guide shaft (14, 16; 80; 90; 100) comprises an intracorporal portion (23) having positioning means for pivotable fixation of the image pick-up unit (20), **characterized in that that** the positioning means have a holder (21; 81; 101) pivotably fixed at the intracorporal portion (23) of the guide shaft (14; 60; 80; 90; 100), at which holder (21; 81; 101) the image pick-up unit (20) is arranged in a distance from a location where the holder (21; 81; 101) is linked to the guide shaft (14; 60; 80; 100), such that the image pick-up unit (20) is intracorporally pivotable into a working position, in which the optical axis (30) runs angularly to a longitudinal center axis (34) of the guide shaft (14; 60; 80; 90; 100) and points to the longitudinal center axis (34).

## Revendications

1. Dispositif permettant de traiter un patient de manière intracorporelle et à invasion minimale, avec un instrument de travail (24) qui peut être introduit dans une cavité corporelle (12) du patient pour accomplir une étape de traitement, une extrémité distale (38) de l'instrument de travail introduit (24) définissant une zone de travail intracorporelle (36), et avec au moins une unité (20) d'enregistrement d'image pour enregistrer une image de la zone de travail intracorporelle (36), avec de plus des moyens de positionnement pour orienter un axe optique (30) de l'unité (20) d'enregistrement d'image en fonction d'une position spatiale de la zone de travail intracorporelle (36), les moyens de positionnement contenant une tige de guidage (14 ; 60 ; 80 90; 100) dans laquelle est guidé l'instrument de travail (24), et l'unité (20) d'enregistrement d'image étant fixée de manière pivotante sur une section intracorporelle (23) de la tige de guidage (22, 42, 44 ; 22, 62, 64), **caractérisé en ce que** les moyens de positionnement présentent un support (21 ; 81 ; 101) fixé pivotant sur la section intracorporelle (23) de la tige de guidage (14 ; 60 ; 80 ; 90 ; 100), sur lequel l'unité (20) d'enregistrement d'image est placée à distance d'un point d'articulation du support (21 ; 81 ; 101) sur la tige de guidage (14 ; 60 ; 80 ; 100), de telle manière que l'unité (20) d'enregistrement d'image peut pivoter de manière intracorporelle jusqu'à une position de travail dans laquelle l'axe optique (30) s'étend en direction d'un axe longitudinal médian (34) de la tige de guidage (14 ; 80) en formant un angle avec celui-ci.

2. Dispositif selon la revendication 1; **caractérisé en ce que** le support présente au moins un bras pivotant (22 ; 84 ; 104) fixé de manière articulée à la section intracorporelle.

3. Dispositif selon la revendication 2, **caractérisé en ce que** l'unité (20) d'enregistrement d'image est placée à l'extrémité libre du dit au moins un bras pivotant (104), de telle manière que l'axe optique (30) de l'unité (20) d'enregistrement d'image s'étend environ perpendiculairement à l'axe longitudinal (106) du bras pivotant (104) et est tourné vers l'axe longitudinal médian (34) de la tige de guidage (100).

4. Dispositif selon la revendication 3, **caractérisé en ce que** le bras pivotant (104) présente une longueur réglable.

5. Dispositif selon l'une des revendications 1 à 4, **caractérisé en ce que** le support présente un mécanisme d'articulation (22 ; 82) à plusieurs axes.

6. Dispositif selon l'une des revendications 1 à 5, **caractérisé en ce que** l'unité (20) d'enregistrement d'image peut pivoter jusqu'à une position de repos sur la tige de guidage (60 ; 80), dans laquelle un contour extérieur de section (72) de l'unité (20) d'enregistrement d'image est placé pour l'essentiel en recouvrement avec un contour extérieur de section (74) de la tige de guidage (60 ; 80).

7. Dispositif selon l'une des revendications 1 à 6, **caractérisé en ce que** l'unité (20) d'enregistrement d'image peut être fixée à la tige de guidage (90) par l'intermédiaire d'un mécanisme de couplage (94) activable de façon intracorporelle.

8. Dispositif selon l'une des revendications 1 à 7, **caractérisé en ce que** les moyens de positionnement comprennent un couplage mécanique forcé entre l'instrument de travail (24) et l'unité (20) d'enregistrement d'image.

9. Dispositif selon la revendication 8, **caractérisé en ce que** les moyens de positionnement présentent des moyens d'arrêt (110) pour une immobilisation axiale au moins partielle de l'instrument de travail (24) par rapport à la tige de guidage (100).

10. Dispositif selon la revendication 9, **caractérisé en ce que** les moyens d'arrêt (110) sont conformés de telle manière que l'instrument de travail (24) est mobile librement dans le sens axial par rapport à la tige de guidage (110), à l'intérieur de limites prédéfinies (116, 118), mais entraîne la tige de guidage (100) dans le cas d'un déplacement dépassant les limites axiales prédéfinies (116, 118).

11. Dispositif selon la revendication 8, **caractérisé en ce que** l'instrument de travail (24) est mobile librement dans le sens axial par rapport à la tige de guidage (14), et **en ce que** le support (21) présente des moyens de couplage (42, 44) qui peuvent être mis en prise avec l'instrument de travail (24) de telle manière que, lors d'un déplacement relatif entre l'instrument de travail (24) et la tige de guidage (14), le support (21) est pivoté afin de rapprocher l'axe optique (30) de la zone de travail (36).

12. Dispositif selon l'une des revendications 1 à 11, **caractérisé en ce que** les moyens de positionnement comprennent une unité d'actionnement (62) pour faire pivoter l'unité (20) d'enregistrement d'image, ainsi qu'une unité de capteur (64) couplée à elle, avec laquelle une position actuelle de la zone de travail (36) peut être déterminée.

13. Dispositif selon la revendication 12, **caractérisé en ce que** l'unité de capteur (62) comprend des moyens de mesure afin de déterminer une position relative de l'instrument de travail (24) par rapport à la tige de guidage (60).

14. Dispositif selon la revendication 12 ou 13, **caractérisé en ce que** l'unité de capteur comprend des moyens de traitement d'image pour l'identification de l'extrémité distale de l'instrument de travail sur l'image enregistrée.

15. Dispositif selon l'une des revendications 1 à 14, **caractérisé en ce que** l'axe optique (30) forme dans la position de travail un angle (32) d'au moins 10°, de préférence compris entre 20° et 70°, avec l'axe longitudinal médian (34) de la tige de guidage (14).

16. Dispositif selon l'une des revendications 1 à 15, **caractérisé en ce qu'**une ouverture d'entrée d'image (79) de l'unité (20) d'enregistrement d'image (20) se trouve dans la position de travail à une distance latérale de la tige de guidage (14 ; 60 ; 80 ; 90 ; 100) qui est supérieure à environ 1 cm.

17. Dispositif selon l'une des revendications 1 à 15, **caractérisé en ce que** l'unité (20) d'enregistrement d'image est une sonde vidéo intégrée qui délivre un signal électrique d'image de la zone de travail (36).

18. Dispositif selon la revendication 17, **caractérisé en ce que** l'image enregistrée par l'unité (20) d'enregistrement d'image est transmise par télémétrie.

19. Dispositif selon la revendication 18, **caractérisé en ce que** l'unité (20) d'enregistrement d'image présente un émetteur dont les signaux d'image émis sont reçus par un récepteur.

20. Dispositif selon la revendication 19, **caractérisé en ce que** le récepteur ou au moins son antenne est placé sur la section intracorporelle (23) de la tige de guidage (14 ; 60 ; 80 ; 90 ; 100).

21. Dispositif selon l'une des revendications 18 à 20, **caractérisé en ce que** sur l'unité d'enregistrement d'image est placé un dispositif d'éclairage qui présente de préférence au moins une diode lumineuse.

22. Dispositif selon l'une des revendications 18 à 21, **caractérisé en ce que** l'unité d'enregistrement d'image présente une source d'énergie, par exemple une pile ou un accumulateur pour son alimentation.

23. Dispositif selon l'une des revendications 1 à 22, **caractérisé en ce que** l'unité d'enregistrement d'image est un élément optique qui délivre un signal optique d'image de la zone de travail.

24. Dispositif selon l'une des revendications 1 à 23, **caractérisé en ce que** la tige de guidage (14 ; 60 ; 80 ; 90) présente un canal de guidage (76) ouvert des deux côtés pour recevoir et guider des instruments de travail (24) interchangeables.

25. Dispositif selon l'une des revendications 1 à 24, **caractérisé en ce que** l'instrument de travail (24) est fixé dans la direction radiale dans la tige de guidage.

26. Dispositif selon l'une des revendications 1 à 19, **caractérisé en ce que** l'instrument de travail (24) peut tourner librement autour de son axe longitudinal dans la tige de guidage (14 ; 60 ; 80 ; 90 ; 100).

27. Dispositif selon l'une des revendications 1 à 26, **caractérisé en ce que** l'unité d'enregistrement d'image (20) présente des moyens (26) pour modifier une section d'image enregistrée.

28. Dispositif selon l'une des revendications 1 à 27, **caractérisé en ce qu'**un dispositif d'éclairage (77) est placé sur la section intracorporelle (23) de la tige de guidage (60).

29. Tige de guidage destinée à être utilisée dans un dispositif permettant de traiter un patient de manière intracorporelle et à invasion minimale, un instrument de travail (24) pouvant être introduit dans le dispositif pour accomplir une étape de traitement dans une cavité corporelle, et le dispositif présentant une unité (20) d'enregistrement d'image présentant un axe optique (30), la tige de guidage (14 ; 60 ; 80 ; 90 ; 100) présentant une section intracorporelle (23) qui présente des moyens de positionnement pour fixer de manière pivotante l'unité (20) de réception d'image, **caractérisé en ce que** les moyens de positionnement présentent un support (21 ; 81 ; 101), disposé et pouvant pivoter sur la section intracorporelle (23) de la tige de guidage (14 ; 60 ; 80 ; 90 ; 100), sur lequel l'unité (20) d'enregistrement d'image est placé à distance d'un point d'articulation du support (21 ; 81; 101) sur la tige de guidage (14 ; 60 ; 80 ; 90 ; 100), de telle manière que l'unité (20) d'enregistrement d'image peut pivoter de façon intracorporelle jusqu'à une position de travail dans laquelle l'axe optique (30) s'étend en direction d'un axe longitudinal médian (34) de la tige de guidage (14 ; 60; 80 ; 90 ; 100) en formant un angle avec celui-ci.
